(19) Europäisches Patentamt
European Patent Office
Office européen des brevets

(11) Publication number: **0 079 592 A2**

(12) # EUROPEAN PATENT APPLICATION

(21) Application number: 82110431.2

(22) Date of filing: 11.11.82

(51) Int. Cl.$^3$: C 07 C 29/15
C 07 C 29/32, C 07 C 45/29
C 07 C 51/12

(30) Priority: 12.11.81 US 320587

(43) Date of publication of application: 25.05.83 Bulletin 83/21

(84) Designated Contracting States: DE FR GB IT NL

(71) Applicant: UNION CARBIDE CORPORATION
Old Ridgebury Road
Danbury Connecticut 06817(US)

(72) Inventor: Fiato, Rocco Anthony
275 Country Club Lane
Scotch Plains New Jersey 07076(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) **Catalyst stabilization.**

(57) A process for stabilization of the metal-atom catalyst used in the production of organic chemicals by the reaction of synthesis gas.



Croydon Printing Company Ltd.

Patentanwälte
Wuesthoff-v. Pechmann
Behrens-Goetz
Schweigerstr.
8000 Mü.



# CATALYST STABILIZATION

## BACKGROUND AND SUMMARY OF THE INVENTION

The catalytic reaction of synthesis gas, which is a mixture of carbon monoxide and hydrogen, and of carbon monoxide are well known phenomena used to produce a wide variety of organic chemicals via homologation, hydroformylation or carbonylation processes. In such processes the catalyst generally contains a metal-atom based compound and the catalyst can be either insoluble in the reaction mixture, heterogeneous catalysis, or soluble in the reaction mixture, homogeneous catalysis. In either case it is important to maintain catalytic efficiency and to maintain the catalyst in its active form.

A frequently encountered problem is the loss of catalyst activity, generally caused by precipitation or plating-out of one or more of the metal-atom based componenets present in the catalyst system; for example, it is known that cobalt, ruthenium, palladium platinum, nickel and chromium atom components tend to loose activity for these reasons. When this occurs, reaction rate, selectivity, or both are affected adversely. Though attempts have been made to improve catalyst stability, much still needs to be done; the invention defined in this specification describes one manner in which improvement is attained.

This invention relates to a means for improving the stability of the metal-atom catalysts that are employed in the homologation, hydroformylation and carbonylation reactions of

synthesis gas for the production of organic chemicals. Stabilizaton is achieved by the addition of a polycarboxylic acid compound, as more fully described hereinafter, to the catalyst-containing reaction mixture. The catalyst system generally contains a mixture of one or more metal-atom compounds, a halide promoter and a ligand and/or solvent; in many instances other additives are also present.

Among the many references in which such systems are disclosed one can mention U.S. 2,534,018 issued to W.F. Gresham on December 12, 1950, U.S. 2,535,060 issued to W.F. Gresham on December 26, 1950, U.S. 2,549,470 issued to B.W. Howk et al. on April 17, 1951, U.S., 2,636,046 issued to W.F. Gresham on April 21, 1953, U.S. 3,248,432 issued to A.D. Riley et al. on April 26, 1966, U.S. 3,285,948 issued to C.N. Butler et al. on November 15, 1966, U.S. 3,356,734 issued to Kuraishi et al. on December 5, 1967, U.S. 3,387,043 issued to M. Kuraishi et al. on June 4, 1968, U.S. 2,490,488 issued to S.G. Stewart on December 6, 1949, U.S. 3,972,952 issued to R.T. Clark on August 3, 1976, U.S. 4,111,837 issued to P.D. Taylor on September 5, 1978, U.S. 4,122,110 issued to A. Sugier et al. on October 24, 1978, U.S. 4,133,966 issued to W.R. Pretzer et al. on January 9, 1979, U.S. 4,151,208 issued to Pretzer et al. on April 24, 1979, U.S. 4,162,262 issued to P. C. Ellgen et al. on July 24, 1979, U.S. 4,168,391 issued to W. E. Slinkard et al. on September 18, 1979, U.S. 4,170,605 issued to R.C. Williamson et al. on October 9, 1979, U.S. 4,190,729 issued to D. Fasten on February 26, 1980, U.S. 4,225,517 issued to Gave on September 30, 1980, U.S. 4,233,466 issued

to R.A. Fiato on November 11, 1980 and U.S. 4,277,634 issued to W.E. Walker on July 7, 1981. Also of interest are Japanese patent Publications 77/136110 and 77/136111 to Saito, published November 14, 1977 and European Patent Applications 10,373, and 650023/36, both assigned to British Petroleum.

Another European Patent Application is Publication Number 0,028,474, published May 13, 1981, assigned to Mitsubishi Chemical Company. In this latter publication there is disclosed a five-step process for the production of ethanol, acetic acid and vinyl acetate from methanol, carbon monoxide and hydrogen. The invention defined involves (a) hydrocarbonylation of methanol, carbon monoxide and hydrogen using a catlyst system containing cobalt or ruthenium, a halide or halogen and an organic reaction accelerator to produce ethanol, acetaldehyde, methyl acetate and dimethylacetal; (b) the carbonylation of methyl acetate to acetic anhydride using a catalyst system containing palladium, iridium, rhodium, nickel or cobalt, a halide or halogen, and an organic or inorganic accelerator; (c) the production of ethylidene diacetate and methyl acetate from the dimethylacetal, acetaldehyde and acetic anhydride of steps (a) and (b) using an acid catalyst; (d) the thermal decomposition of the ethylidene diacetate of step (c) to produce vinyl acetate and acetic acid; and the recycle of methyl acetate of steps (a), (c) or (d) to step (b). The publication discloses the use of polycarboxylic acid compounds as accelerators for the hydrocarbonylation and carbonylation reactions of steps (a) and (b). However, there is no recognition, suggestion, or disclosure that such

compounds can have a stabilizng effect on the catalyst per se, nor do any of the specific examples show the use of a polycarboxylic acid compound.

## DETAILED DESCRIPTION OF THE INVENTION

In the catalytic homologation, hydroformylation and carbonylation reactions of synthesis gas to produce organic chemicals, there are three significant parameters or criteria by which the catalysts are judged; namely stability, activity and selectivity. Stability relates to the period of time the catalyst remains active or functional before either breaking down or losing catalytic effect, e.g., by decomposition, precipitation or plating-out; activity relates to the amount of reactants the catalyst converts per unit of time; and selectivity relates to the quantity of desired products produced, as compared to undesired products produced, during the catalytic reaction. The most desirable situation is to have high values for all three parameters; however, this is seldom achieved. It has often been observed that improvement in one of the parameters often tends to have a detrimental effect on one or more of the other parameters and the ultimate result is often an overall less efficient process.

The present invention is directed to the improvement of the stability of the metal-atom catalyst. The improved stability is achieved wtihout essentially any disadvantage to the other parameters. In the process of this invention the metal-atom containing catalyst systems used in the homologation, formylation and carbonylation reactions of synthesis gas to produce organic

chemicals are stabilized by the addition thereto during the reaction of a stabilizing amount, sufficient to stabilize the metal-atom catalyst, of a polycarboxylic acid compound. The discovery that the polycarboxylic acids would have a stabilizing affect on the catalyst was a completely unexpected and unobvious finding and, to the best of our knowledge, has not heretofore been suggested or disclosed. The manner in which this effect is achieved is not fully understood; however, the results achieved are real.

The most commonly observed form of catalyst instability is precipitation or the plating-out of the metal-atom component on the reactor vessel walls. To assess the amount of instability the precipitate can be recovered and weighed or if the catalyst had plated-out on the reactor walls it can be scraped off and weighted. The most desirable situation is that in which the catalyst neither precipitates nor plates-out.

The polycarboxylic acids that can be used as stabilizers in the process of this invention are (i) the aromatic polycarboxylic acids of the general formula:

$$AR(COOH)_x$$

in which Ar is phenyl or naphthyl and x has a value of from 2 to 4, preferably 2, and (ii) the aliphatic polycarboxylic acids of the general formula:

$$XC_mH_{2m}X$$

wherein X is (a) a $-COOH$ group,

(b) a $-N(C_nH_{2n}COOH)_2$ group,

or (c) a $-\underset{\substack{|\\ C_nH_{2n}COOH}}{N}-C_mH_{2m}N(C_nH_{2n}COOH)_2$ group

m has a value of from 2 to 4, preferably 2

or 3; and n has a value of from 1 to 5, preferably from 2 to 3; or the anhydrides of (i) or (ii). These compounds can contain substituents, such as alkyl, nitro, acetyl, aloxy, sulfo, amido or halo groups, that will not unduly interfere with the stabilizing effect of the polycar- boxylic acid or of the synthesis gas reaction. They are well known and illustrative thereof one can mention phthalic acid, isophthalic acid, terephthalic acid, hemimellitic acid, trimellitic acid, mellophanic acid, maleic acid, malonic acid, succinic acid, glutaric acid, adipic acid, ethylenediamine tetraacetic acid, propylenediamine tetraacetic acid and diethylene- triamine pentaacetic acid.

A typical homologation reaction is the reaction of methanol with synthesis gas to produce the higher homologues, e.g., ethanol, a typical hydroformylation reaction is the reaction of methanol with synthesis gas to produce acetaldehyde, and a typical carbonylation reaction is the production of acetic acid by the reaction of methanol with carbon monoxide. All of these reactions are preferably carried out in contact with selected catalysts and under different and selected reaction conditions, all of which are well knwon to one of ordinary skill in the art. The patents previously referred to illustrate only a few of the known basic variations. As is seen, in many instances a trivalent Group V compound is present as a component of the system, often this is a phosphine. It has now also been found that the amount of phosphine charged can be reduced or completely replaced by the polycarboxylic acid compounds and that the metal-atom compound does not

precipitate. The reduction or elimination of the phosphine compound is advantageous because of the high cost of such compounds. Also present in the processes described in the cited references are the metal-atom compounds such as compounds of cobalt, ruthenium, molybdenum, palladium, platinum, osmium, chromium, nickel, iron, vanadium and manganese. Of these, cobalt is the metal most frequently cited. It is these metal-atom compounds that have a tendency to precipitate or plate-out, as a consequence of which the reaction is deleteriously affected. Another component generally present in the catalytic reaction is an halogen compound, for example, an iodide or elemental iodine.

In order to simplify matters, the homologation reaction for the production of ethanol will be used to describe the invention.

In the homologation process of this invention to produce ethanol, in a laboratory reactor, the reactants, namely methanol, a cobalt compound as the catalyst, a halogen promotoer such as iodine or an iodide compouund, a ruthenium compound and the polycarboxylic acid stabilizer are added to the reactor. The reactor is sealed and purged with carbon monoxide and then a carbon monoxide-hydrogen gas mixture is added to a desired pressure. The reactor and contents are heated to a specified temperature and the pressure and temperature are maintained for the desired time.

A problem often encountered in synthesis gas reactions has been the precipitation or plating-out of the metal-atom catalyst component. This is undesirable in any reaction since upon its occurrence catalytic activity is significantly

reduced or completely lost. When this occurs the usefulness of the catalyst is limited to a single batch or pass-through, preventing continuous prôcêss operation and commercial acceptance. The instant invention overcomes this problem to a significant degree permitting the process to be operated continuously.

The ruthenium component of the system can be supplied from any number of sources and those skilled in the art are fully familiar with themany classes of ruthenium compounds that can be used to supply the ruthenium component. The ruthenium complexes which catalyst the reaction are not specifically known; they can be mono-ruthenium or poly-ruthenium complexes. Thus, any of the ruthenium compounds such as the ruthenium salts, oxides, carbonyls, organic carboxylic acid salts, or ruthenium metal itself, which may from soluble ruthenium carbonyl or hydrocarbonyl compounds under the reaction conditions can be used. Among the ruthenium compounds that can be used as the source for the ruthenium component one can mention ruthenium dioxide, ruthenium sesquioxide, ruthenium tetraoxide, ruthenium trichloride or tribromide or triiodide, ruthenium acetate, ruthenium acetylacetonate, ruthenium propionate, ruthenium octanoate, ruthenium pentacarbonyl, triruthenium dodecacarbonyl, ruthenium carbonyl hydride, diruthenium nonacarbonyl, ruthenium (2,4-pentanedionate), or any other ruthenium compound which can generate a soluble ruthenium complex under the reaction conditions. They can be used individually or as mixtures of two or more ruthenium compounds.

The cobalt component of the system can come from a number of sources such as any of known cobalt carboxylates such as cobalt formate, cobalt acetate, cobalt propionate, cobalt butyrate, cobalt valerate, cobalt hexanoate, and the like; the known cobalt carbonyl compounds such as dicobalt octacarbonyl, methyl cobalt tetracarbonyl, acetyl cobalt tetracarbonyl, and the like, or their phosphine substituted analogs many of which are known to those skilled in the art; cobalt oxide and cobalt hydroxide; cobalt carbonate and cobalt bicarbonate; and the soluble cobalt halides such as cobalt iodide, cobalt bromide and cobalt chloride.

Any of the known suitable halogen compounds can be used, elemental iodine or the iodides being preferred. Illustrative thereof one can mention iodine, potassium iodide, tetramethylammonium iodide, tetramethylsulfonium iodide, methyl iodide, butyl iodide, tetrabutylammonium iodide, hydrogen iodide, tetramethylphosphonium iodide, cesium iodide, tetraethylammonium iodide, cobalt iodide and bromine and its corresponding bromides. Further, one can use mixtures of two or more such halogen compounds. Those skilled in the art identify the halogen compounds by the term halogen promoters. In addition to the halogen promoters, one can also include any of the other promoters known in the art, such as the Lewis bases (e.g., $R_3N$, $R_3P$, $R_2S$ onium type compounds where R is an organic group, and zinc iodide).

One can also have present in the reaction mixture any of the solvents known to those skilled in the art as useful. There are so well known that no further identification is required here.

The mole ratio of hydrogen ($H_2$) to carbon monoxide in the synthesis gas can range from 0.1:1 to 10:1, preferably from 1:1 to 5:1 and most preferably from 2:1 to 3:1.

The whole catalyst system, which is the metal-atom catalyst components plus the halogen promoter and the polycarboxylic acid stabilizer, is present in an amount which is from 1 to 20 weight percent of a liquid (e.g., methanol) reactants introduced into the reactor, preferably from 8 to 12 weight percent thereof.

The mole ratio of cobalt to methanol can be from 1:5 to 1:5,000, preferably from 1:50 to 1:500.

The mole ratio of cobalt to ruthenium can be from 1:0.003 to 1:3, preferably from 1:0.03 to 1:0.3.

The mole ratio of cobalt to polycarboxylic acid stabilizer can be from 1:0.1 to 1:100, preferably from 1:1.5 to 1:10.

The mole ratio of polycarboxylic acid stabilizer to total halide content can be from 1:0.001 to 1:250, preferably from 1:0.36 to 1:5.

The mole ratio of cobalt to total halide can be from 1:0.1 to 1:25, preferably from 1:1 to 1:5.

The improved catalytic reaction of this invention is carried out at a temperature of from about 100°C to 350°C, preferably from 160°C to 280°C.

The reaction can be carried out at total
34,5 bar 1 380 bar
pressures of from 500 psi to 20,000 psi or more;
69 bar 828 bar
preferably from 1,000 psi to 12,500 psi; and most
172,5 bar 552 bar
preferably from 2,500 psi to 8,000 psi. Variations is pressure can affect the rate and selectivity to ethanol or other selectively desired product and

those skilled in the art can readily ascertain by routine procedures the best conditions to employ with each particular catalyst, promoter and solvent system employed. While higher pressures tend to result in greater product- ivities, they may not be justified economically since they require higher captial investment and since, generally, good rates can be achieved at the lower pressures indicated.

In the following examples catalyst stability was determined gravimetrically. When the catalyst was unstable the cobalt precipitated or plated-out on the walls of the reaction vessel and the amount was determined by scraping the deposit off the walls and weighing it. In the examples, when the amount of deposited cobalt material recovered in this fashion was from 150 to 250 mg or more, the experiment was given a rating of 3, this generally corresponded to the deposition of about 2/3 or more of the charged cobalt. When the amount of deposited cobalt was 150 mg or less, the experiment was given a rating of 2. When none of the cobalt deposited, the experiment was given a rating of 1.

the following examples serve to further illustrate the invention. In the examples time and other reaction conditions were maintained essentially constant throughout the series in order that valid comparisons could be made. The data confirms the heretofore unknown discovery that the defined polycarboxylic acids stabilize the metal catalyst used in sythesis gas reactions.

Example 1

A glass-lined 500 ml rocking autoclave was

charged with 50 ml of reagent grade methanol, 4 millimoles of cobalt (235 mg Co) as cobaltous acetate tetrahydrate, 0.44 millimoles of ruthenium as ruthenium chloride, 4 millimoles of iodide as iodine and 4 millimoles of N,N,N',N'-ethylenediamine tetraacetic acid as stabilizer. The reactor was purged with carbon monoxide and then pressurized to 276 bar 4,000 psig with a synthesis gas mixture having a 2:1 molar ratio of hydrogen to carbon monoxide and sealed. The reactor and its contents were weighed, heated to 175°C and reacted for two hours during which time the reactor was rocked to achieve thorough mixing. At the end of this period the reactor was cooled to room temperature, weighed and vented. The liquid reaction mixture was recovered and analyzed. The interior walls were examined for deposited cobalt and none was found; nor was any cobalt deposit found in the liquid reaction mixture.

The liquid reaction mixture was analyzed using a vapor phase gas chromatograph equipped with a thermal conductivity detector and a 3,175 mm one-eighth inch by 1,8 m six feet column packed with ten weight percent of polyethylene glycol having an average molecular weight of about 10,000 supported on diatomaceous earth, comparing results obtained with those from standard solution; and in accord with standard procedures in this technology.

In a similar manner the reaction is carried out using N,N,N',N'-propylenediamine tetraacetic acid as the stabilizer.

Example 2

In a manner similar to that described in Example 1, methanol was reacted with synthesis gas.

The conditions and concentrations of compounds charged to th autoclave were as described in Example 1 with the exception that the N,N,N',N'-ethylenediamine tetracetic acid was replaced by 4 millimoles of N,N,N',N',N''-diethylenetriamine pentaacetic acid. As in Example 1, no cobalt deposit was found.

Example 3

In a manner similar to that described in Example 1, methanol was reacted with synthesis gas. The conditions and concentrations of compounds charged to the autoclave were as described in Example 1 with the exception that the N,N,N',N'-ethylenediamine tetracetic acid was replaced by 8 millimoles of succinic acid.

Example 4

In a manner similar to that described in Example 1, methanol ws reacted with synthesis gas. The conditions and concentrations of compounds charged to the autoclave were as described in Example 1 with the exeption that the N,N,N',N'-ethylenediamine tetracetic acid was replaced by 8 millimoles of phthalic acid.

In a similar manner the reaction is carried out using glutaric acid as the stabilizer.

For comparative purposes, two control experiments were carried out; one in which the stabilizer was completely absent and the second in which a monobasic acid, propionic acid, was used as a replacement for the polycarboxylic acid stabilizers used in Examples 1 through 4.

Control A

This control experiment was carried out

following the same procedure used in Example 1. However, the experiment did not contain the stabilizer N,N,N',N'-ethylenediamine tetracetic acid. Upon completion of the reaction it was found that essentially all of the cobalt catalyst had deposited on to the reactor walls.

Control B

The procedure used in the control experiment was as described in Example 1, but substituting propionic acid for the N,N,N',N'-ethylenediamine tetracetic acid. At the conclusion of the experiment cobalt metal was recovered from the walls of the reactor.

The results of Examples 1 through 4 and Controls A and B are summarized in Table I. As is evident, significant or complete catalyst decomposition was observed in the two control experiments and no loss of catalyst was observed in Examples 1 through 4, which examples are representative of this invention.

TABLE I

| | % Methanol Conversion | % Ethanol Selectivity | Catalyst Stability Rating |
|---|---|---|---|
| Ex. 1 | 38 | 52 | 1 |
| Ex. 2 | 40 | 41 | 1 |
| Ex. 3 | 33 | 55 | 1 |
| Ex. 4 | 45 | 59 | 1 |
| Cont. A | 43 | 62 | 3 |
| Cont. B | 48 | 53 | 2 |

## CLAIMS

1. In a process for the production of organic chemicals from synthesis gas or carbon monoxide wherein the reaction is catalyzed by metal-atom compound, the improvement comprising adding to the reaction mixture a stabilizing amount sufficient to stabilize the catalyst of a catalyst stabilizer selected from the group consisting of:

(i) an aromatic polycarboxylic acid of the general formula:

$$AR(COOH)_x$$

wherein Ar is a substitued or unsubstituted phenyl or naphthyl group and x has a value of from 2 to 4, or (ii) a substituted or unsubstituted aliphatic polycarboxylic acid of the general formula:

$$XC_mH_{2m}X$$

wherein X is (a) a $-COOH$ group,

(b) a $-N(C_nH_{2n}COOH)_2$ group;

or (c) a $-N(C_nH_{2n}COOH)-C_mH_{2m}N(C_nH_{2n}COOH)_2$ group

m has a value of from 2 to 4 and n has a value of from 1 to 5.

2. A process as claimed in claim 1, wherein the metal-atom compound is a cobalt compound.

3. A process as claimed in claim 2, wherein there is additionally present a halogen promoter and/or a ruthenium compound and a halogen promoter.

4. A process as claimed in claim 1-3, wherein x has a value of 2, m has a value of from 2 to 3 and n has a value of from 2 to 3.

5. A process as claimed in claim 1-4, wherein Ar is phenyl.

6. A process as claimed in claim 1-5, wherein said catalyst stabilizer is phthalic acid or succinic acid.

7. A process as claimed in claim 1-6, wherein X is a carboxyl group or a $-N(C_nH_{2n}COOH)_2$ group or a $-\underset{\substack{| \\ C_nH_{2n}COOH}}{N}-C_mH_{2m}N(C_nH_{2n}COOH)_2$ group.

8. A process as claimed in claim 1-6, wherein said catalyst stabilizer is N,N,N',N'-ethylenediamine tetraacetic acid or N,N,N',N',N"-diethylenetriamine pentaacetic acid.

9. A process as claimed in claim 1, wherein such synthesis gas process is a homologation process or a hydroformylation process or a carbonylation process.